# EUROPEAN PATENT APPLICATION

(11) **EP 3 467 842 A1**
(43) Date of publication of application: **10.04.2019**
(21) Application number: 17195528.9
(22) Date of filing: 09.10.2017
(51) Int. Cl.: G16H 50/50, G06T 17/00

(54) **METHOD FOR SIMULATING FOOT AND/OR ANKLE**

(71) Applicant: Digital Orthopaedics, 1435 Mont-Saint-Guibert (BE)
(72) Inventor: DELEU, Paul-André, 1200 Woluwe-Saint-Lambert (BE); FERRÉ, Bruno, 34300 Agde (FR); LEEMRIJSE, Thibaut, 1200 Woluwe-Saint-Lambert (BE); STENTI, Andréa, 1000 Bruxelles (BE); HALIOUA, Éric, 1180 Uccle (BE)
(74) Representative: Savoye, Anne

(57) **Abstract**

The present invention relates to a computer-implemented method for simulating foot and/or ankle pathologies. The present invention further relates to evaluation of treatment of a foot and/or ankle disorder on a subject.

## Description

### FIELD OF INVENTION

The present invention pertains to the field of numerical simulation of foot and/or ankle in relation to the lower limb. In particular, the invention relates to the evaluation of the risk connected to a conservative and/or surgical treatment for pathologies of foot and ankle.

### BACKGROUND OF INVENTION

The foot and ankle is a highly complex structure generally composed of 28 bones, synovial joints, more than hundreds of ligaments and muscles which forms the kinetic linkage allowing the lower limb to interact with the ground, a key requirement for gait and other activities of daily living. The disorders of the foot and ankle attend a large part of the population and may be treated by conservative treatment or by surgery.

In daily practice, the assessment of foot and lower limb function and of the mechanical pathogenesis of foot and lower limb disease mostly relies on observation, anamnesis, palpation, clinical assessment, goniometry, medical imaging, muscle testing and in rare cases on gait analysis. Based on the results of this plethora of tests, clinicians define a conservative and/ or a surgical treatment with respect to clinical guidelines to restore and maintain proper bony alignment and muscle balance. Despite improvement of clinical and radiographic parameters, a growing body of evidence suggests that the approach consisting in treating all patients presenting the same pathology with a same treatment does not always ensure a complete restoration of function. Inter-subject anatomical heterogeneity is one of the main reason why this may be the case and thus precludes the above cited approach to properly treat foot and lower limb diseases. Numerous studies pointed out the existence of anatomical foot variations in terms of joint surfaces and geometries, muscles insertion points, presence of accessory muscles or ligaments, ligaments insertion points and their potential impact on the mechanical behavior of the foot and the lower limb. This further underpins that the same approach for different patients could potentially induce different contact pressure at the joints due to the large existence of inter-subject anatomical heterogeneity.

Although the diagnostic techniques can be useful in the clinical practice, they can hardly be used as a rationale for the evaluation of surgical interventions since their metric properties and quality appraisal does not reflect objectively the functional capacity of the patients. These traditional assessments are more intended to identify a specific pathology or impairment, but do not necessarily identify dysfunction or pathological stress distribution in the bones and soft tissue and the contact pressure at the joints. Therefore, rather than continue to apply a poorly founded conservative and/or surgical model of foot type whose basis is to make all feet criteria for the anatomical/radiological "ideal" or "normal" foot, it would be of greater interest to incorporate anatomical variation between feet anatomies and identify patient's proper mechanical reference. Significant inter-subject anatomical variation exists and question any conservative and/or surgical notion that is based on the concept of a single ideal anatomical/radiological foot type and alignment.

Nowadays, there is a growing interest for the development of realistic visual model with numerical modelling of physical tissue properties allowing the surgeon to access to a realistic 3D display of the patient-specific surgery. The personalized medicine is a powerful tool to define optimal patient-specific treatments. Computational models have been developed as tools to study the biomechanics of human body. The information generated by the numerical model may be used to select and to plan the most suitable treatment for a specific patient.

EP 2 471 483 describes a computer implemented method for automatically planning of a surgical procedure providing a virtual model of the body part of the patient that necessitate surgery.

WO 2012/021894 describes a method that uses patient-specific information gathered preoperatively in conjunction with optimization algorithms to determine an optimal implant design and an optimal positioning for its implantation into the particular patient's joint. The three-dimensional model reconstructing the geometry, the shape, the relevant surfaces and other morphological aspects of the patient's anatomy, created from the imaging data of the patient, is used to define the optimal implant for that particular patient.

Those methods provide patient specific treatment solution from computation modelling of the patient anatomy but they do not allow to confront between different possible solutions, i.e. between an invasive surgical treatment and a conservative treatment or the evaluation of the effectiveness of the treatment on the patient.

Moreover, the foot/ankle complex is a complicated joint. The foot and ankle is made up of the twenty-eight individual bones of the foot, together with the long-bones of the lower limb to form a total of thirty-three joints. Although frequently referred to as the "ankle joint", there are a number of articulations which facilitate motion of the foot. The ankle joint complex is made up of the talocalcaneal (subtalar), tibiotalar (talocrural) and transverse-tarsal (talocalcaneonavicular) joint and is composed of the talus, fibula, and tibia, the last of which bears 85% of the weight pressing down on the foot during standing. This joint allows for dorsiflexion and plantar flexion (up-and-down motion). The talus is also part of the ankle's subtalar joint, a synovial joint that rests below the ankle joint that allows for side-to-side motion (inversion and eversion). The foot complex comprises the forefoot, the midfoot and the hind foot. The forefoot is composed of the five phalanges and their connecting metatarsals. Each phalanx is made up of several small bones. The big toe (also known as the hallux) has two phalanx bones and has one joint, called the interphalangeal joint. The big toe articulates with the head of the first metatarsal and is called the first metatarsophalangeal joint. Underneath the first metatarsal head are two tiny, round bones called sesamoids. The other four toes each have three bones and two joints. The phalanges are connected to the metatarsals by five metatarsal phalangeal joints at the ball of the foot. The forefoot bears half the body's weight and balances pressure on the ball of the foot. The midfoot has five irregularly shaped tarsal bones, forms the foot's arch, and serves as a shock absorber. The bones of the midfoot are connected to the forefoot and the hindfoot by muscles and the plantar fascia (arch ligament). Finally, the hindfoot is composed of three joints and links the midfoot to the talus. The top of the talus is connected to the two long bones of the lower leg (tibia and fibula), forming a hinge that allows the foot to move up and down. The heel bone (calcaneus) is the largest bone in the foot. It joins the talus to form the subtalar joint. The bottom of the heel bone is cushioned by a layer of fat.

When standing, the ground reaction forces (GRF) acting on feet are evenly distributed between both feet, and those forces are equal in magnitude to the body weight. Break into a walk or run, though, and the math changes: in addition to countering the vertical force of gravity, feet contend with friction and other horizontal forces of physics as one pushes off and moves forward. The ankle joint complex bears a joint force of approximately five times body weight during stance in normal walking, and up to thirteen times body weight during activities such as running.

The key movement of the ankle joint complex are plantar- and dorsiflexion, occurring in the sagittal plane; ab-/adduction occurring in the transverse plane and inversion-eversion, occurring in the frontal plane. Combinations of these motions across both the subtalar and tibiotalar joints create three-dimensional motions called supination and pronation.

Degenerative processes of the foot and ankle, such as post-traumatic osteoarthritis, may also have a significant impact on the biomechanical function of the ankle. Post-traumatic osteoarthritis is the most prevalent osteoarthritis type of the ankle joint. Moreover, in comparison to the hip and knee surgery, problems remain after ankle surgical interventions: slower walk, reduced ankle ROM, ankle moments and power compared to healthy controls.

Whilst actual gait analysis can be used as an objective tool for quantifying motion of lower limb joints and forces that act upon these joints, it cannot separate the talocalcaneal (subtalar), tibiotalar (talocrural) and transverse-tarsal (articulation of Chopart and articulation of Lisfranc) joint due to the major limitation of accurately measuring talus motion using skin-mounted markers.

Therefore, one of the major issues in the present context is the development of a method allowing the accurate modelling of the patient anatomy in order to simulate different treatment strategies and provide a plurality of outputs allowing the surgeon to choose the one treatment strategy associated with the low risk of relapse.

### SUMMARY

According to one aspect the invention relates to a simulation method for determining at least an area of interest of a foot and/or an ankle of a subject, the method comprising:
a) receiving at least one anatomical and/or functional image relating to at least a foot and a related ankle of the subject; and
b) determining at least one simulation instruction;
c) determining, from said at least one image, at least two segmented volumes corresponding to at least one anatomical portion of said foot and/or ankle;
d) determining at least one kinematic of at least two of the segmented volumes;
e) generating a multibody and/or finite element three-dimensional model of the foot and/or ankle using the at least two segmented volumes and/or the at least one kinematic;
f) selecting a simulation mode according to the at least one simulation instruction, said simulation mode comprising at least one force vector representing a mechanical stress, a frequency, a number and a duration of application of said mechanical stress;
g) simulating the model of the foot and/or ankle according to the simulation mode selected; and
h) generating from the simulation a repartition of quantitative values, said quantitative values representing the resultant of the forces of said at least one mechanical stress.

The step of receiving at least one anatomical and/or functional image relating to at least one foot and a related ankle of the subject may be, according to one embodiment, a step of receiving in a processor information concerning the subject, including information relating to the anatomy of at least a foot and a related ankle of the subject.

The step of determining at least one simulation instruction may be, according to one embodiment, a step of receiving in a processor information relating to subjective parameters evaluated by the subject.

The steps of:
c) determining, from said at least one image, at least two segmented volumes corresponding to at least one anatomical portion of said foot and/or ankle;
d) determining at least one kinematic of at least two of the segmented volumes; and
e) generating a multibody and/or finite element three-dimensional model of the foot and/or ankle using the at least two segmented volumes and/or the at least one kinematic;
may be, according to one embodiment, a step of using, in a processor, the received information concerning the specific subject for generating at least one subject-specific model of the foot and ankle in relation with the lower limb, comprising bones and soft tissues.

The steps of:
f) selecting a simulation mode according to the at least one simulation instruction, said simulation mode comprising at least one force vector representing a mechanical stress, a frequency, a number and a duration of application of said mechanical stress; and
g) simulating the model of the foot and/or ankle according to the simulation mode selected;
may be, according to one embodiment, a step of simulating the at least one subject-specific foot and ankle model in an at least one static condition and/or in an at least one dynamic condition.

According to one embodiment, the step of determining at least two segmented volumes corresponding to at least one anatomy portion of said foot and/or ankle extracted from said at least one image, comprises the segmentation of a volume of different types for example bones, tendons, ligaments, articular cartilages and other soft tissues, each type of volume comprising one or more volumes. For example, a tendon may be modelled by two volumes, a bone volume may be modelled by *n* volumes and a ligament by *p* volumes.

According to one embodiment, the method further comprises:
- determining a corrective instruction, said corrective instruction defining a corrective parameter for at least one segmented volume and/or at least one kinematic and/or at least one simulation instruction.

According to one embodiment, the step of determining a corrective instruction comprises receiving in a processor further information concerning the subject leading to a diagnosis, including:
I. information generated by a clinical decision support system; and/or
II. information generated by a quantitative functional analysis of the foot and lower limb.

The step of determining a corrective instruction releases on selecting at least one value from a predefined list of set of values.

According to one embodiment, said list is a list of predefined corrective instructions, each predefined corrective instruction corresponding to a corrective scenario each corrective scenario comprising a set of corrective parameters.

According to one embodiment, a predefined corrective instruction of the list is a model, also called in the detailed description pathological model.

According to another aspect the present invention relates to a method for subject-specific simulation of foot and/or ankle, the method comprising:
a) receiving in a processor information concerning the subject, including:
   I. information relating to the anatomy of at least a foot and a related ankle of the subject; and
   II. information relating to subjective parameters evaluated by the subject;
b) in the processor, using the received information concerning the specific subject, generating at least one subject-specific model of the foot and ankle in relation with the lower limb, comprising bones and soft tissues;
c) in the processor simulating the at least one subject-specific foot and ankle model in an at least one static condition and/or in an at least one dynamic condition;
d) outputting from the processor a set of information obtained from the simulation of the at least one subject-specific foot and ankle model.

According to one embodiment, wherein the subject presents a pathology of the foot and/or ankle and the method comprises the further steps
- a step a.2): receiving in a processor further information concerning the specific subject leading to a diagnosis, including:
   I. information generated by a clinical decision support system; and/or
   II. information generated by a quantitative functional analysis of the foot and lower limb;
- a step a.3): receiving in the processor information concerning the specific subject defined by a user choice of at least one pathology model related to the diagnosis obtained in the step a.2).

According to one embodiment, the subject-specific foot and ankle model is a multibody and/or finite element three-dimensional model.

According to one embodiment, the multibody and/or finite element three-dimensional subject-specific foot and ankle model comprises:
a) a modelling of the totality of the foot and ankle bones;
b) a modelling of the articular cartilage between bones of the foot and ankle;
c) a modelling of the tibia, the fibula and the talus or another part of the foot;
d) a modelling of ligaments, tendons and plantar fascia; and
e) a modelling of the soft tissues volume of the foot and ankle surrounding the models of the foot bones.

According to one embodiment, the subject-specific foot and ankle model includes the modelling of interaction due to the contact between the external soft tissues with a ground, the interaction due to the contact between the soft tissues surface and/or the bones and the interactions due to the contact between bones in joints.

According to one embodiment, the step of receiving information concerning the subject comprises receiving information relating to at least one of the following: a lifestyle of the subject, at least one physiological attribute of the subject, a demographic characterization of the subject, an earlier injury of the subject, a comorbidity condition of subject, an imaging information, a quantified functional data of the subject and a bone strength characterization of the patient.

According to one embodiment, the step of receiving information generated by a quantitative foot and lower limb function analysis concerning the subject comprises receiving information relating to biomechanical static and/or dynamic characteristics.

According to one embodiment, the step of receiving information relating subjective parameters evaluated by the subject comprises receiving information relating to at least a type of physical activity and performances level the subject desires to attend.

According to one embodiment, the output parameters of the computer implemented method are graphically represented to be visualized on a display.

According to one embodiment, the at least one parameter computed in step d) is a ground reaction force divided by the surface of contact of the foot with the ground or the scalar value of the stress submitted by the soft-tissues in proximity of the bones.

According to one embodiment, the at least one parameter computed in step d) is the amplitude of rotation or translation of the joint, the pressure on the bones contact surfaces or articular cartilage surfaces or the stress on all tendons and ligaments.

According to one embodiment, the method further comprises:
- a step a.4): receiving in a processor information concerning a user choice of at least treatment model for the subject-specific model;
- a step c.2): simulating the at least one treatment model chosen for the subject-specific foot and ankle model, generating at least one after-treatment model of the subject-specific foot and ankle;
- a step c.3): simulating the after-treatment subject-specific foot and ankle model in an at least one static condition and/or in an at least one dynamic condition;
- a step d.2): outputting from the processor a set of information for evaluating the at least one simulated treatment;
wherein the step c) as defined in the hereabove described embodiment is optional.

According to one embodiment, the at least one treatment is a conservative treatment or a surgical treatment.

According to one embodiment, the output set of information comprises parameters evaluating the risks of recurrence associated to the at least one simulated treatment and/or practice parameters for the at least one simulated treatment.

According to one embodiment, the parameters evaluating the risks of the at least one simulated treatment comprise intra-articular changes of pressure and tissue stress during a specific task.

According to one embodiment, the practice parameters for the at least one simulated treatment comprise surgical planning report, parameters for 3D printed patient specific solutions and robotic surgical plan.

The present invention further comprises a system for simulation of foot and/or ankle comprising a data processing system comprising means for carrying out the steps of the method according to the embodiment described hereabove.

The present invention further comprises the computer program product for simulation of foot and/or ankle comprising instructions which, when the program is executed by a computer, cause the computer to carry out the steps of the method according to embodiment described hereabove.

The present invention further comprises computer-readable storage medium comprising instructions which, when the program is executed by a computer, cause the computer to carry out the steps of the method according to the embodiments hereabove.

### DEFINITIONS

- **"Functional activities parameters"** refers to the measurable parameters and factor obtained by a functional analysis of a subject, such as for example gait analysis of the subject. The parameters may comprise step length, stride length, cadence, speed, dynamic base, progression line, foot angle, hip angle, squat performance and like. In one embodiment, the functional activities parameters include gait parameters.
- **"Soft tissues"** refers to the tissues that connect, support or surround other structures not being hard tissues as bone, such as articular cartilage, tendons, ligaments, fascia, skin, fibrous tissues, fat, and synovial membranes, muscles, nerves and blood vessels.
- **"Foot and/or ankle in relation with the lower limb"** refers to the ensemble comprising a foot, an ankle and the associated lower limb below the knee.
- **"Pathology of the foot and/or ankle"** may be replaced in the present invention by the term "impairment of the foot and/or ankle".

### BRIEF DESCRIPTION OF THE DRAWINGS

**Figure 1** shows a flow chart schematically illustrating one non-limiting example of a method for simulating a foot and/or ankle of a specific subject.
**Figure 2** is a flow chart showing a non-limiting example of a method for simulating a model of a subject's foot and/or ankle in relation with the lower limb in a static and/or dynamic condition.
**Figure 3** is a flow chart showing a non-limiting example of a method for simulating at least one treatment model for a subject's foot and/or ankle and evaluate it after-treatment in a static and/or dynamic condition through simulation. The after-treatment subject-specific foot and ankle model is evaluated over a period of time with a loop that may be repeated n times.
**Figure 4** is a flow chart showing a non-limiting example of a method for simulating at least one treatment model for a subject's foot and/or ankle in relation with the lower limb. If the treatment model is evaluated as effective, the method ends. Otherwise a new treatment model may be simulated for the subject-specific foot and ankle.
**Figure 5** shows the grey-scale-bar graph that may be used to visually represents the output information.
**Figure 6** is a grey-scale coding graph showing the tissue stress distribution during motion in hard and soft tissues.

### DETAILED DESCRIPTION

This invention relates to a method for subject-specific simulation of foot and/or ankle. According to one embodiment, the method of the present invention is computer implemented.

According to an embodiment, the method of the invention comprises:
a) receiving in a processor information concerning the subject;
b) in the processor, using information received in steps a), generating at least one subject specific model of the foot and ankle in relation with the lower limb of the subject; and
c) in the processor simulating the at least one subject-specific foot and ankle model, generated in step b), in an at least one static condition and/or in an at least one dynamic condition;
d) outputting from the processor a set of information obtained from the simulation of step c).

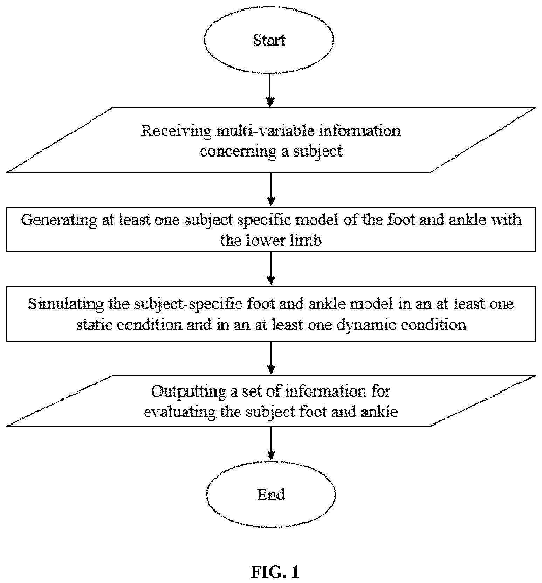

The flow chart of the method according to this embodiment is represented in Figure 1.

According to embodiment, the subject does not present any pathology of the foot and/or ankle.

According to an embodiment, the step of receiving in a processor information concerning the subject includes:
I. information relating to the anatomy of at least a foot and an ankle with the lower limb of the subject; and
II. information relating to subjective parameters evaluated by the subject.

According to one alternative embodiment, the subject presents a pathology of the foot and/or the ankle.

According to this embodiment, the step of receiving in a processor information concerning the subject further includes information leading to a diagnosis. According to an embodiment, information leading to a clinical assessment of the foot and ankle includes:
I. information generated by a clinical decision support system; and/or
II. information generated by a quantitative analysis of the foot and lower limb function.

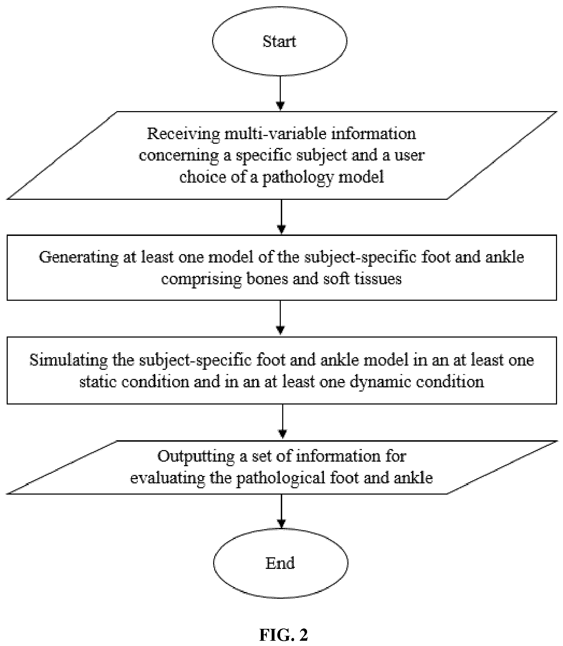

According to an embodiment, receiving in a processor information concerning a user choice includes at least one pathology model related to the diagnosis obtained in the step a), from a predefined list of pathologies' choices. The flow chart of the method according to this embodiment is represented in Figure 2.

The schematic flowchart diagrams in the Figures illustrate the functionality and operation of possible implementations of methods and computer program products according to various embodiments of the present invention. In this regard, each block in the schematic flowchart diagrams may represent a module, segment, or portion of code, which comprises one or more executable instructions of the program code for implementing the specified logical function(s).

It should also be noted that, in some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. Other steps and methods may be conceived that are equivalent in function, logic, or effect to one or more blocks, or portions thereof, of the illustrated Figures.

Although various arrow types and line types may be employed in the flowchart, they are understood not to limit the scope of the corresponding embodiments. Indeed, some arrows or other connectors may be used to indicate only the logical flow of the depicted embodiment. For instance, an arrow may indicate a waiting or monitoring period of unspecified duration between enumerated steps of the depicted embodiment. It will also be noted that each block and combinations of blocks flowchart diagrams, can be implemented by special purpose hardware-based systems that perform the specified functions or acts, or combinations of special purpose hardware and computer readable program code.

The method according to the present invention comprises a preliminary step of receiving in a processor information concerning the subject. According to one embodiment, receiving information concerning the subject comprises receiving information relating to at least one of the following: the demographic characterization of the subject (including age, gender, race, body mass index, hereditary disorders, etc.), the lifestyle of the subject (such as for example diet, body mass index, smoking or not, sporty or not), the medical history of the subject (such as for example an earlier injury, hereditary disorders, earlier surgeries, etc.), at least one physiological attribute of the subject, quantified functional activities parameters of the subject, information about injured stress tissue, the comorbidity condition of subject, the bone strength characterization of the patient, pain index and annotation, pain location, results of clinical analysis.

According to one embodiment, information concerning the subject includes information relating to the anatomy of at least a foot and ankle. In one embodiment, information relating to the anatomy of at least a foot, an ankle and a related lower limb of the subject includes, but is not limited to, information defining at least in part soft tissues.

According to one embodiment, information relating to the anatomy of at least a foot, an ankle and a related lower limb of the subject is obtained from imaging data. According to an embodiment, the information relating to the anatomy of at least a foot, an ankle and a related lower limb of the subject is a static and/or a dynamic information. By means of non-limiting examples, the imaging data concerning the at least one foot, the ankle with respect to the lower limb may be acquired by two-dimensional (2D) imaging technics such as radiography, ultrasounds, fluoroscopy, laser scan, photography, and the like; or by tri-dimensional (3D) imaging technics such as magnetic resonance imaging, computer tomography, laser scan, and the like. According to a particular embodiment, information relating to the anatomy of at least a foot and an ankle of the subject are dynamic information. According to an embodiment, dynamic information includes 3D imaging data combined with motion and/or 4D imaging data which comprising imaging data in combination with motion type information such as in the functional analysis.

According to one embodiment, information concerning the subject further includes information relating to subjective parameters. According to one embodiment, subjective parameters are evaluated by the subject. According to one embodiment, said information relating to subjective parameters comprises subject-specific requirements, such as information relating to at least a type of physical activity (also herein referred as "task") and/or performances level. By means of non-limiting examples, the physical activity may be any type of sport like walking, running, jumping, hiking, cycling, gymnastics, dancing, football, racquet sports, rock climbing, grappling, equine sports, golf, skiing, sailing, hockey, hurling and shinty, lacrosse, polo, swimming, weight lifting and others. According to one embodiment, the physical activity may be daily activities, and performance levels may be the ease to carry out these daily tasks. According to one embodiment, the level of performance desired by the subject may be selected from a list comprising low, medium and high level. According to another embodiment, the sporting level desired by the subject may be selected from a list comprising beginner, semi-professional and professional level. The subjective parameters may include an assessment of working capacity, accordingly for instance to the International Classification of Functioning, Disability and Health; impairment rating representing a measure of daily living performances (i.e. Barthel ADL index) and/or a disability rating (i.e. American Medical Association guidelines).

According to one embodiment, said subjective parameters is associated for the simulation to a simulation instruction comprising the following: at least one force vector representing a mechanical stress, a frequency, a number and a duration of application of said mechanical stress. For example, a subjective parameter such as a professional football player willing to play for the next 10 years is associated to a simulation instruction comprising for example a scenario comprising the application of a combination of n vectors, each vector been applied at a point located at the interface between two volumes, the frequency of applied mechanical stress. Said scenario may be repeated at a frequency *p* with a duration of *d* wherein said frequency and duration are extracted from the predefined information. In the present example, the predefined information is qualification of "professional player" and "10 years".

According to the embodiment wherein the subject does present a foot and/or ankle pathology, the processor generates at least one subject-specific model of the foot and ankle in relation with the lower limb, comprising bones and soft tissues using the received information relating to the anatomy of at least a foot and a related ankle of the subject and information relating to subjective parameters evaluated by the subject.

According to the embodiment wherein the subject presents a pathology of the foot and/or the ankle, information concerning the subject further includes information leading to a diagnosis. According to one embodiment, information leading to a diagnosis includes information generated by a knowledge-based or a non-knowledge based clinical decision support system. Information may be further generated by a quantitative foot and lower limb function analysis or further received from objective clinical assessment.

According to one embodiment, the non-knowledge-based clinical decision support system, which is an assistance with clinical decision-making tasks for the physician, provides at least the most likely diagnosis. According to one embodiment, the non-knowledge-based clinical decision support system uses a machine learning algorithm, which allows to improve the systems performances by learning from past experiences and/or find underlaying patterns in clinical data. The non-knowledge-based clinical decision support system can be implemented by an artificial neural networks, genetic algorithms or support vector machine.

According to one embodiment, the knowledge-based clinical decision support, which is an assistance with clinical decision-making tasks for the physician, provides at least the most likely diagnosis on the base of subjective information provided by the subject and by the objective clinical assessment and/or observation made by a health care professional. The knowledge-based clinical decision support may further use information provided from data bases comprising information from medical literature and/or medical experts. According to one embodiment, the knowledge-based clinical decision support uses a Bayesian reasoning.

According to one embodiment, the clinical decision support system provides one diagnosis. According to one embodiment, the clinical decision support system provides more than one diagnosis, also called potential diagnoses. According to one embodiment, the potential diagnoses provided by clinical decision support system are associated with probability for each diagnosis. According to one embodiment, steps b) to d) of the method of the invention are carried out for the preferred diagnosis or the most likely diagnosis. According to one embodiment, method steps b) to d) of the present invention are carried out for each potential diagnosis separately. According to one embodiment, the information provided to the clinical decision support are inserted manually by the user by means of a keyboard or a transfer from a storage memory or by means of an artificial intelligence using voice pattern recognition and interaction system or voice-driven personal medical assistant or artificial intelligence powered decision support technology. According to one embodiment, the information used from the clinical decision support system are demographic data, pain location, subjective representation of the pathology and results of pathology-specific objective test, such as, by means of no-limiting examples, passive restricted range of motion test using Tinel's foot test, Morton's test, Mulder's test, Hintermann's test, too many toes sign, foot posture index, Thompson test, drawer test, Coleman block test and the like.

According to one embodiment, the information generated by a quantitative functional analysis of the foot and lower limb comprises biomechanical characteristics. Said biomechanical characteristics may comprise a biomechanical reference frame, loading bearing axes and other dynamic parameters such as the kinematics and kinetics of the foot and lower limb, plantar pressure measurements, joint references, joint loading, joint kinematics data (displacement, velocity, accelerations, etc.) and kinetics data (forces, torques, intra-articular pressure, etc.), inverse and forward dynamics, electromyographic and acoustic myographic signals of the foot and lower limb at rest or in motion or information concerning the activities of the subject in the daily routine or combinations thereof. The biomechanical characteristics may further comprise information concerning ground reactions (forces and torques, impulses, peak pressure values, peak pressure ratio, pressure time integral, peak pressure gradient, load rate, pressure contact area, force-time integral and integral ratios, power ratio, center pressure, etc.), strain and stress measurements of soft tissues (articular cartilage, ligaments, tendons, muscles, etc.) and hard tissues, geometry and shape of tissues, measures concerning muscle function and physiological cost, spatio-temporal parameters (step length, stride length, foot angle of gait, walking speed, cadence, velocity, step time, stride time, single support, double support, swing time, etc.) and alignment of an implant with respect to the anatomy of the subject. According to one embodiment, the biomechanical characteristics are measured using technics such as three-dimensional stereophotogrammatric analysis, force platforms, pedobarographic, plantar pressure platform, electromyography, kinesiologic electromyography, accelerometers, gyroscopes or other technics. According to one embodiment, information leading to a diagnosis includes information generated by a clinical decision support system combined to information generated by a quantitative foot and lower limb function analysis.

According to the embodiment wherein the subject presents a pathology, the method according to the present invention comprises a further step consisting in receiving in a processor information concerning a user choice. According to one embodiment, said choice concerns at least one pathology model related to the at least one diagnosis obtained from the clinical decision support system and/or the information generated by a quantitative functional analysis of the foot and lower limb. According to one embodiment, the at least one pathology model is chosen from a predefined list of pathologies' models. According to one embodiment, the list of pathologies' models comprises all musculoskeletal pathologies located at the foot and ankle. The list of pathologies may comprise different type of trauma such as malleolar fractures, tibial pilon fractures, calcaneus fractures, navicular and midfoot injuries and metatarsal and phalangeal fractures, arthritis of the ankle joint and the joints of the hindfoot (tarsals), midfoot (metatarsals) and forefoot (phalanges), congenital and acquired deformities including adult acquired flatfoot, non-neuromuscular foot deformities, diabetic foot disorders, hallux valgus and several common pediatric foot and ankle conditions such as clubfoot, flat feet, tarsal coalitions, etc.

The method according to the present invention is capable of supporting highly complex pathologies interesting foot and ankle. This represents a major improvement respect to the methods of the prior-art, which were exclusively directed to pathologies concerning the less complex articulations of knees and hip.

According to one embodiment, the method, further comprises a step of, generating at least one model of the pathological foot and ankle using the received information concerning the specific subject and the at least one pathology model. According to one embodiment, the at least one model of the pathological foot and ankle comprises bones and soft tissues (articular cartilage, ligaments, tendons, muscles, nerves, etc.). The "model of the pathological foot and ankle" may be understood more generally as "subject-specific foot and ankle model" as described in the following description.

According to one embodiment, the subject-specific foot and ankle model is a multibody and/or finite elements three-dimensional model. According to one embodiment, for the generation of the subject-specific foot and ankle model, the imaging data of the subject are segmented obtaining information on the bones geometry, soft tissue placement and anatomical characteristics.

According to one embodiment, the multibody and/or finite element three-dimensional subject-specific foot and ankle model comprises a modelling of the totality of the foot and ankle bones, a modelling of the articular cartilage between bones of the foot and ankle, a modelling of the tibia and the fibula or another part of the foot, a modelling of the ligaments, tendons and the plantar fascia and a modelling of a foot and ankle soft tissues volume surrounding the computer modelled foot bones, tibia and fibula, ligaments and the plantar fascia. According to one embodiment, in the model the proximal, medial and distal phalanges are fused to obtain a simplified model for the foot fingers. According to one embodiment, tendons are or are not included in the model. According to one embodiment bones, ligaments and soft tissues material properties are subject-specific and are obtained from imaging data or other technics data (MRI, ultrasounds etc.). According to one embodiment, bones, ligaments, articular cartilage, tendons, plantar fascia and soft tissues material properties are taken from literature or medical data bases. According to one embodiment, the multibody and/or finite element three-dimensional subject-specific foot and ankle model comprises exclusively a modelling of the totality of the foot bones and a modelling of the tibia and the fibula with respect to the lower limb.

According to one embodiment, the multibody and/or finite element three-dimensional subject-specific foot and ankle model comprises a modelling of the totality of the foot bones, a modelling of the tibia and the fibula, a modelling of the ligaments and the plantar fascia. Those simplifications of the multibody and/or finite element three-dimensional subject-specific foot and ankle model could be used to reduce the computation time.

According to one embodiment, the three-dimensional subject-specific foot and ankle model includes the modeling of interaction due to the contact between the external soft tissues with a ground and the interaction due to the contact between the soft tissues surface, the bones and the constraints inside the bones and between the bones including articular cartilages.

According to one embodiment, the three-dimensional subject-specific foot and ankle model includes the modelling of interaction due to the contact between the external soft tissues with a ground, the interaction due to the contact between the soft tissues surface and the bones, the interactions due to the contact between bones in joints and any other interaction at the interface between different tissues.

According to the embodiment wherein the subject presents no pathology, the method comprises a step of simulating the at least one subject-specific foot and ankle model in an at least one static condition and/or in an at least one dynamic condition.

According to one embodiment, the simulation of at least one static condition comprises the step of choosing a simulation mode and simulating the action of at least one mechanical stress on the three-dimensional subject-specific foot and ankle model, wherein the mechanical stress have the characteristics specified in the simulation mode. The mechanical stress may be for example applied on at least one of the segmented volume of the subject-specific foot and ankle model in order to simulated the effect of body weight, when a ground surface is simulated in contact with at least one segmented volume representing the sole of the foot. According to this example the mechanical stress may be represented by a vector aligned along a vertical direction, oriented through the foot and having magnitude proportional to the body weight of the subject. According to this example, the repartition of quantitative values is the spatial distribution of the ground reactions.

According to one embodiment, the simulation of at least one dynamic condition comprises the step of choosing a simulation mode and simulating the action of at least one mechanical stress and at least one kinematics on the three-dimensional subject-specific foot and ankle model.

According to the embodiment wherein the subject presents a pathology, the user choice further concerns the choice of at least one treatment model for the pathological model. According to one embodiment, the at least one treatment model is chosen from a predefined list of treatments models, on the base of the information received in the preliminary step.

According to one embodiment, the at least one treatment model is a model of conservative treatment or surgical treatment. According to one embodiment, the list of conservative treatment models comprises medications or injections, such as nonsteroidal antiinflammatory drugs or steroids; BOTOX, Silicone, etc.; orthotics (especially altered shoes), cane, or boot walker for functional mobilization; physical therapy to restore function, strength, and movement; platelet-rich plasma injections; and shockwave or Extracorporeal Pulse Activation Therapy (EPAT) directed into the soft tissue.

According to one embodiment, the list of surgical gesture models comprises the models of simulating osteosynthesis surgery, simple or complex articular fusion, prosthesis insertion, ligamentoplasty, tendon transfer and osteotomy. According to one embodiment, multiple surgical gesture models are selected and combined together. According to one embodiment, the choice of at least one surgical treatment comprises the selection from a predefined library of at least one implant and/or at least one surgical instrument.

The method according to the present invention may comprise a further step consisting in simulating in the processor the at least one treatment for the pathological foot and ankle model generated in the step above. According to one embodiment, the at least one treatment model chosen for the subject-specific foot and ankle model is simulated, generating at least one after-treatment model of the pathological foot and ankle. According to one embodiment, the at least one the after-treatment subject-specific foot and ankle model is simulated in at least one static condition and/or in at least one dynamic condition.

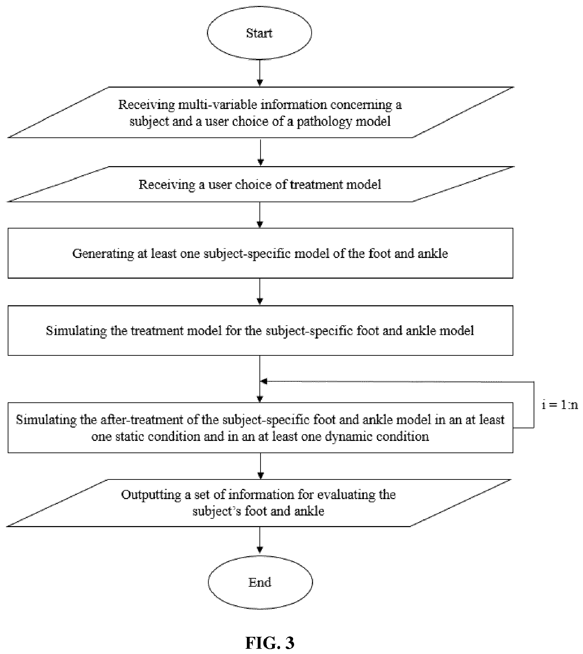

The reliability or the impact of the surgery is simulated in order to estimate the survival rate. This is a quality measure to avoid creating secondary pathologies due to the surgery. According to one embodiment, the impact of surgery is evaluated simulating the evolution of the subject-specific foot and ankle model, after treatment, over a specified period of time. In one embodiment, the after-treatment period of time simulated is more than one month, preferably more than six months, more preferably more than one year. In one embodiment, the after-treatment period simulated is a period of more than two, three, four or five years. The reliability or the impact of the surgery may be evaluated simulating the degradation of biological tissues or implantation material over the specified period of time. The evolution over a time period of the after-treatment foot and ankle model may be obtained by repeating for a number for times n the step of simulation in static and/or dynamic condition, as shown in the flow chart of the method in Figure 3.

According to one embodiment, the at least one static and/or dynamic condition is selected from the subject-specific requirements. As a non-limitative example, the treatment is simulated for a football activity at a semi-professional level over a period of five years.

According to one embodiment, more than one treatment is simulated. According to one embodiment, when several treatments may be considered for at least one pathology user choice, the method of the invention comprises the simulation of each treatment.

The step of simulating the treatment according to subject-specific data and subject-specific requirements such as a desired task, allows to predict the failure of the treatment after a specified period of time, such as for example mechanical failure of an implant after two years of running. Therefore, if several treatments may be considered, the step of treatment simulation may help the user or the clinician to make an informed choice.

An advantage of the present method is the prediction and analysis of internal stress and strain distributions acting on all the anatomical structures of the foot and ankle during static and dynamic conditions.

Another advantage of the present invention is the implementation of preventive strategies to reduce the functional limitations linked to an impairment and to avoid secondary pathologies to the adjacent and non-adjacent joints, bone and/or soft tissues.

The method according to the present invention comprises a final step of outputting from the processor a set of information generated during the simulation step. According to one embodiment, the set of output information are generated during the simulation of the at least one foot and ankle with respect to the lower limb model. According to one embodiment, the set of output information are generated during the simulation of the at least one subject-specific foot and ankle model. According to one embodiment, the set of output information are generated during the simulation of the at least one treatment simulated for the at least one subject-specific foot and ankle model in the step above.

The set of output information computed may comprise at least one of the following parameters: the axis and the amplitude of rotation or translation of the joint, the pressure on the bones contact surfaces or the stress on all tendons and or ligaments and other biomechanical parameters. Said biomechanical parameters may comprise a biomechanical reference frame, loading bearing axes and other dynamic parameters such as the kinematics and kinetics of the foot and lower limb, plantar pressure measurements, joint references, joint loading, joint kinematics data (displacement, velocity, accelerations, etc.) and kinetics data (forces, torques, intra-articular pressure, etc.), inverse and forward dynamics, electromyographic and acoustic myographic signals of the foot and lower limb at rest or in motion or information concerning the activities of the subject in the daily routine or combinations thereof. The biomechanical parameters may further comprise information concerning ground reactions (forces and torques, impulses, peak pressure values, peak pressure ratio, pressure time integral, peak pressure gradient, load rate, pressure contact area, force-time integral and integral ratios, power ratio, center pressure, etc.), strain and stress measurements of soft tissues (ligaments, tendons, articular cartilage etc.) and hard tissues, geometry and shape of tissues, measures concerning muscle function and physiological cost, spatio-temporal parameters (step length, stride length, foot angle of gait, walking speed, cadence, velocity, step time, stride time, single support, double support, swing time, etc.) and alignment of an implant with respect to the anatomy of the subject. According to one embodiment, information leading to a diagnosis includes information generated by a clinical decision support system combined to information generated by a quantitative foot and lower limb function analysis.

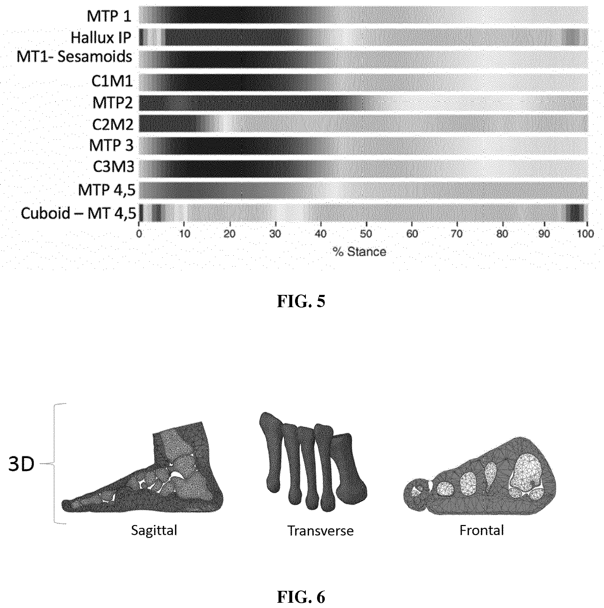

According to one embodiment, said set of output information comprises parameters evaluating the risks of the at least one simulated treatment. According to one embodiment, the output information comprises "a prediction and analysis of the risk of impairment recurrence of foot or ankle due to mechanical failure" and/or "an evaluation of the work capacity of a subject performing a specific dynamic task". According to one embodiment, the output information comprises the best set of therapeutic (conservative or surgical) parameters where the global musculoskeletal system of the foot and lower limb is the least stressed by mechanical forces during one or more predefined set of functional activities. The best set of therapeutic solution may comprise a surgical and a conservative treatment, for example a surgery combined with an adequate post-operative conservative treatment. According to one embodiment, parameters evaluating the risks of the at least one simulated treatment comprise intra-articular changes of pressure during a specific task and/or tissue stress during a specific task. By means of non-limiting example, said risk evaluation parameters may be graphically represented with easily interpretative graph such as the color coding graphs, such as color-bar graph, or radar charts to display multivariate data. Figure 5 shows an example of a color-bar graph that may be used. Figure 6, provide a 3D visual representation through a color coding graph of the mechanical stress distribution during motion in bones and soft tissues.

According to one embodiment, the output information comprises a risk score for the at least one simulated treatment. In one embodiment, the risk score may be a score out of ten, twenty, fifty or one hundred. In one embodiment, the risk score may be percentage of risk, with a healthy foot/ankle as a reference value (i.e. representing 100%). The risk may be a score using as reference a generic healthy lower limb model or the reference model may be selected from a data base as the one being more similar to the actual subject model. The risk may be scored as well using the subject model of the foot and ankle with respect to the lower limb wherein all the pathological (or impairment) parameters and mechanical stress have been removed.

According to one embodiment, said set of output information further comprises parameters concerning the at least one treatment for the foot and ankle of the subject. According to one embodiment, parameters are practice parameters, such as for example surgical planning report, parameters for 3D printed patient specific solutions (implants, patient specific instruments, cutting guides, patient-specific implant positioner, etc.) or robotic surgery. Examples of conservative treatment parameters include, but are not limited to, rehabilitation parameters and 3D printed patient specific solutions such as orthotics, in particular orthopedic shoes or insoles, braces, prosthesis, smart footwear and others. Examples of surgical treatment parameters include, but are not limited to, surgical planning report, in particular a planning customized to surgeon's requirements, 4D imaging data containing tissues stress data, 5D imaging data comprising motion, robotic surgery and 3D printed patient specific solution such as implants, specific surgical instruments and others.

According to one embodiment, the output information comprises a risk prediction of the mechanical failure of implants dedicated to the surgical treatment of foot and ankle impairments in a subject.

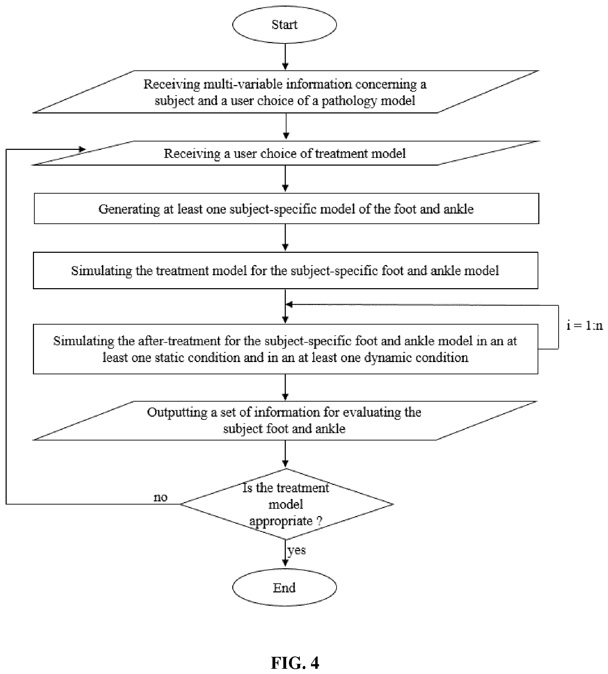

According to one embodiment, if the risk score obtained for the simulated treatment is associated to an elevated risk of recurrence, another treatment model may be chosen by the user to be simulated as shown in the flow chart in Figure 4.

According to one embodiment, the output information comprises an automated choice selecting the optimal treatment for the subject.

According to one embodiment, the output information obtained with the method according to the present invention are communicated to the user thought a web application, a smartphone app or a tablet app.

According to one embodiment, the output information is communicated to the clinician using a screen comprised in a computer device or more specifically a tactile tablet or a smartphone. The output information may be visualized by the clinician or surgeon during treatment procedures using optical head-mounted display or intelligent glasses implementing augmented reality such as Google Glass.

According to one embodiment, the totality of information concerning the subject are pseudo-anonymized before storage into a data server. The subject pseudo-anonymized information may be further encrypted before storage. According to one embodiment, all identification information is removed from the collected data concerning the subject. By means of non-limiting example, the name and surname of the subject are delated from all computation tomography and magnetic resonance imaging DICOM file or others imaging or text files and replaced with a unique identification code provided by a third-party partner. All pseudo-anonymized information is stored by the third-party partner in a certified ISO 270001 facility. According to one embodiment, the totality of pseudo-anonymized data is encrypted using a symmetric cryptographic method and stored separately from the symmetric master key that allows the decryption of the subject personal information.

The present invention may further comprise a machine learning module as artificial intelligence.

In general, machine learning is classified into various algorithms such as supervised learning and unsupervised learning according to its target or conditions. The present invention has an object of learning to evaluate the plurality of output parameters and choose upon the simulated treatment strategies the one implying the lower risk of collateral problems. Therefore, the machine learning module implements a supervised learning. According to one embodiment, said machine learning method for evaluating treatment strategies involves training a computer by using a training mode of the machine learning module to construct a transformation function. According to one embodiment, said machine learning module comprises a production mode which uses the transformation function to evaluate the treatment strategies.

In a preferred embodiment, the machine learning is a supervised machine learning method which uses as training examples the multiples medical cases collected into a medical database. According to one embodiment, the machine learning used is preferably an artificial neural network. By means of no-limiting example the neural network algorithms may be a group method of data handling networks, a convolutional neural network, a long short-term memory network, a deep belief network, large memory storage and retrieval neural network, deep Boltzmann machine, stacked (de-noising) auto-encoder, deep stacking network, tensor deep stacking network, etc.

The present invention also relates to a system for evaluation of foot and/or ankle of a subject, the system comprising a data processing system comprising means for carrying out the steps of the method according to anyone of the embodiments described hereabove.

According to one embodiment, the data processing system is a dedicated circuitry or a general purpose computer, configured for receiving the data and executing the operations described in the embodiment described above. According to one embodiment, the data processing system comprises a processor and a computer program. The processor receives digitalized input data and processes them under the instructions of the computer program to compute the simulation of the foot and ankle model. According to one embodiment, the computing device comprises a network connection enabling remote implementation of the method according to the present invention. According to one embodiment, input data are wirelessly communicated to the data processing device. According to one embodiment, the means used to visualize the output information wirelessly receives the output information from the data processing device.

The present invention further relates to a computer program product for evaluation of foot and/or ankle of a subject, the computer program product comprising instructions which, when the program is executed by a computer, cause the computer to carry out the steps of the computer-implemented method according to anyone of the embodiments described hereabove.

Computer program code for carrying out operations for aspects of the present invention may be written in any combination of one or more programming languages, including an object oriented programming language such as Java, Python, Ruby, PHP, C++ or the like and conventional procedural programming languages, such as the "C" programming language or similar programming languages. The computer program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

The computer program code may also be loaded onto a computer, other programmable data processing apparatus such as a tablet or phone, or other devices to cause a series of operational steps to be performed on the computer, other programmable apparatus or other devices to produce a computer implemented process such that the program code which execute on the computer or other programmable apparatus provide processes for implementing the functions/acts specified in the flowchart and/or block diagram block or blocks.

The present invention further relates to a computer-readable storage medium comprising instructions which, when the program is executed by a computer, cause the computer to carry out the steps of the computer-implemented method according to anyone of the embodiments described hereabove.

The present invention further relates to a method for improving understanding of musculoskeletal impairments of the foot and ankle using a subject-specific computational foot and ankle model.

The method according to the present invention may also be used as a tool for education in foot and ankle surgery (orthopedics, podiatry, physiotherapy, and the like).

The method according to the present invention may also be used to quantitatively define clinical and surgical guidelines related to specific foot and ankle pathologies or impairments.

While various embodiments have been described and illustrated, the detailed description is not to be construed as being limited hereto. Various modifications can be made to the embodiments by those skilled in the art without departing from the true spirit and scope of the disclosure as defined by the claims.

## Claims

1. A method for subject-specific simulation of foot and/or ankle, the method comprising:
a) receiving in a processor information concerning the subject, including:
**I.** information relating to the anatomy of at least a foot and a related ankle of the subject; and
**II.** information relating to subjective parameters evaluated by the subject;
b) in the processor, using the received information concerning the specific subject, generating at least one subject-specific model of the foot and ankle in relation with the lower limb, comprising bones and soft tissues;
c) in the processor simulating the at least one subject-specific foot and ankle model in an at least one static condition and/or in an at least one dynamic condition;
d) outputting from the processor a set of information obtained from the simulation of the at least one subject-specific foot and ankle model.

2. The method according to claim **1,** wherein the subject presents a pathology of the foot and/or ankle and the method comprises the further steps:
- a step a.2): receiving in a processor further information concerning the specific subject leading to a diagnosis, including:
**I.** information generated by a clinical decision support system; and/or
**II.** information generated by a quantitative functional analysis of the foot and lower limb;
- a step a.3): receiving in the processor information concerning the specific subject defined by a user choice of at least one pathology model related to the diagnosis obtained in the step a.2).

3. The method according to either claim **1** or **2,** wherein the subject-specific foot and ankle model is a multibody and/or finite element three-dimensional model.

4. The method according to claim **3,** wherein the multibody and/or finite element three-dimensional subject-specific foot and ankle model comprises:
a) a modelling of the totality of the foot and ankle bones;
b) a modelling of the articular cartilage between bones of the foot and ankle;
c) a modelling of the tibia, the fibula and the talus or another part of the foot;
d) a modelling of ligaments, tendons and plantar fascia; and
e) a modelling of the soft tissues volume of the foot and ankle surrounding the models of the foot bones.

5. The method according to anyone of claims **1** to **4,** wherein the subject-specific foot and ankle model includes the modelling of interaction due to the contact between the external soft tissues with a ground, the interaction due to the contact between the soft tissues surface and/or the bones and the interactions due to the contact between bones in joints.

6. The method according to anyone of claims **1** to **5,** wherein receiving information concerning the subject comprises receiving information relating to at least one of the following: a lifestyle of the subject, at least one physiological attribute of the subject, a demographic characterization of the subject, an earlier injury of the subject, a comorbidity condition of subject, an imaging information, a quantified functional data of the subject and a bone strength characterization of the patient.

7. The method according to anyone of claims **1** to **6,** wherein receiving information generated by a quantitative foot and lower limb function analysis concerning the subject comprises receiving information relating to biomechanical static and/or dynamic characteristics.

8. The method according to anyone of claims **1** to **7,** wherein receiving information relating subjective parameters evaluated by the subject comprises receiving information relating to at least a type of physical activity and performances level the subject desires to attend.

9. The method according to anyone of claims **1** to **8,** wherein the at least one parameter computed in step d) is the amplitude of rotation or translation of the joint, the pressure on the bones contact surfaces or articular cartilage surfaces or the stress on all tendons and ligaments.

10. The method according to anyone of claims **2** to **9,** further comprising:
- a step a.4): receiving in a processor information concerning a user choice of at least treatment model for the subject-specific model;
- a step c.2): simulating the at least one treatment model chosen for the subject-specific foot and ankle model, generating at least one after-treatment model of the subject-specific foot and ankle;
- a step c.3): simulating the after-treatment subject-specific foot and ankle model in an at least one static condition and/or in an at least one dynamic condition;
- a step d.2): outputting from the processor a set of information for evaluating the at least one simulated treatment;
wherein the step c) as defined in claim **1** is optional.

11. The method according to claim **10,** wherein the at least one treatment is a conservative treatment or a surgical treatment.

12. The method according to anyone of claims **10** to **11,** wherein the output set of information comprises parameters evaluating the risks of recurrence associated to the at least one simulated treatment and/or practice parameters for the at least one simulated treatment.

13. A system for simulation of foot and/or ankle, the system comprising a data processing system comprising means for carrying out the steps of the method according to anyone of claims **1** to **12.**

14. A computer program product for simulation of foot and/or ankle, the computer program product comprising instructions which, when the program is executed by a computer, cause the computer to carry out the steps of the method according to anyone of claims **1** to **12.**

15. A computer-readable storage medium comprising instructions which, when the program is executed by a computer, cause the computer to carry out the steps of the method according to anyone of claims **1** to **12.**
